# EUROPEAN PATENT SPECIFICATION

(11) **EP 2 832 371 B1**
(45) Date of publication and mention of the grant of the patent: **01.02.2017**
(21) Application number: 12873232.8
(22) Date of filing: 12.12.2012
(51) Int. Cl.: A61K 47/22, A61K 9/08, A61K 9/14

(54) **APPLICATION OF GASTRODIN AS SOLUBILIZER AND METHOD FOR PREPARING INJECTION OR POWDER FOR INJECTION**
VERWENDUNG VON GASTRODIN ALS LÖSUNGSMITTEL UND VERFAHREN ZUR HERSTELLUNG EINER INJEKTION ODER EINES INJEKTIONSPULVERS
APPLICATION DE GASTRODINE COMME AGENT DE SOLUBILISATION ET PROCÉDÉ DE PRÉPARATION D'UNE INJECTION OU D'UNE POUDRE POUR INJECTION

(30) Priority: 31.03.2012 CN 201210092802
(43) Date of publication of application: 04.02.2015
(73) Proprietor: KPC Pharmaceuticals, Inc., Kunming, Yunnan 650106 (CN)
(72) Inventor: CHEN, Yunjian, Kunming, Yunnan 650106 (CN); YANG, Zhaoxiang, Kunming, Yunnan 650106 (CN); ZHU, Ze, Kunming, Yunnan 650106 (CN); FANG, Fang, Kunming, Yunnan 650106 (CN); PU, Junxue, Kunming, Yunnan 650106 (CN)
(74) Representative: Bösl, Raphael Konrad
(86) International application number: PCT/CN2012/086445
(87) International publication number: WO 2013/143323

(56) References cited:
- CN-A- 1 682 824
- CN-A- 1 803 182
- CN-A- 1 923 241
- CN-A- 102 335 227
- KR-A- 20080 037 300
- KR-A- 20090 059 270

## Description

### FIELD OF THE INVENTION

The present invention relates to the field of medicine, and particularly relates to the use of gastrodin as a solubilizer and a method for preparing an injection or a powder for injection.

### BACKGROUND OF THE INVENTION

Flavonoid compounds in natural products have structural and bioactive diversity, and have hepatoprotective, anti-inflammatory, estrogenic, antibacterial, antiviral, anti-tumor and anti-oxyradical effects. For example, quercetin, rutin, hyperin, breviscapine, puerarin, as well as total flavone of Pueraria and Ginkgo, etc. all exhibit a protective effect on ischemic brain injury; hyperin, silibinin, luteolin, and total flavone of hippophae, etc. have a protective effect on myocardial ischemia injury; and puerarin, daidzein and total flavone of Ginkgo leaves, etc. can significantly reduce cerebrovascular resistance and myocardium oxygen consumption as well as the generation of lactic acid, and have a significant protective effect on myocardial anoxia injury. Puerarin, daidzein, licoflavone as well as total flavone of Hippophae and Sophora flavescens have an antiarrhythmic effect. Luteolin, quercetin, kaempferide, apigenin as well as 4,2',4',-trihydroxy-3'-isopentenyl chalcone and 7,4'-dihydroxy-3'-isopentenyl chalcone have an inhibitory effect on platelet aggregation and thrombosis caused by arachidonic acid, collagen, platelet-activating factor, thrombin and so on, but these flavonoid compounds generally have lower solubility in water.

There are also many active ingredients in the natural products which belong to carbon glycoside compounds, such as barbaloin, homoorientin, mangiferin, bergenin and aloesin or the like. Generally, these carbon glycoside compounds are very stable to acid and base, and the glycosidic bonds are difficult to be cleaved completely with a chemical method; however, mangiferin and isomangiferin have a carbon glycosidic structure, with lower solubility in various solvents.

There are also an important class of stilbene compounds in the natural products, including diphenylethylene glycosides, diphenylethyl compounds, phenanthrenes and derivatives thereof. The diphenylethylene glycoside compounds mainly have antibacterial, antifungal, hypolipidemic, hepatoprotective, antioxidant, antithrombotic, coronary vessel-expanding, antihypertensive, anti-allergy, platelet aggregation-inhibiting, anti-tumor and anti-HIV and other effects, but also have effects of inhibiting activities of DNA polymerase, antagonism of estrogen and inhibiting the growth of human breast cancer. The activity of the diphenylethyl compounds mainly consists in antibacterial effect, antifungal effect, inhibition of HIV, inhibition of 5-lipoxygenase, inhibition of DNA polymerase β, regulation of troponin and cytotoxic activity. The main bioactivity of the phenanthrene compounds mainly consists in anti-platelet aggregation and anti-tumor effects, which have been reported to have inhibitory effects on liver cancer and Ehrlich ascites carcinoma, and show cytotoxic effects on human cancer cells A549, SK-OV-3 and HL-60. The diphenylethylene glycoside compounds are mainly monomer derivatives or polymers (such as polydatin, triptophenolide, rhapontin and deoxyrhapontin (methyl polydatin)) with resveratrol, piceatannol or hydrides thereof as basic units in structure, which are divided into resveratrol oligomer analogues, isorhapontigenin oligomer analogues, piceatannol oligomer analogues, resveratrol and oxyresveratrol oligomers, glycosides formed by resveratrol oligomers, of which the linking sugars are all glucoses with the number thereof varying from 1 to 3, and the forms of the glycoside are oxyglycoside and glycoside. The above stilbene compounds have lower solubility in water.

There are usually problems of low solubility, low bioavailability for most of the natural products, which to some extent limit the possibility of their development as new drugs. Therefore, one of the main challenges for getting new drugs to the market is to find a suitable method to improve the solubility of poorly soluble drugs and improve the bioavailability.

The drug solubility can be improved by simple means, such as salt-forming, using a mixture solvent, adding a solubilizer and others, but also can be improved by more complex preparation approaches and new technologies. Salt-forming is the most common process to increase the drug solubility. Most of the organic drugs are weak acids or weak bases, which are converted to salts and separated in an organic solution. It is generally unexpected in advance whether a certain salt is stable in water which may be converted to an acid or a base or decomposed, how about its hygroscopicity, how about its solubility under different conditions, whether the toxicity is enhanced, and whether it is suitable for preparing certain preparation and the like. It will be known whether the certain salt is suitable only after pharmacokinetics, pharmacodynamics, toxicity and other tests. When using a mixed solvent to prepare liquid preparations, injections and the like, the mixed solvent is often used to enhance the solubility of the poorly soluble drugs. The most common one is to add alcohols such as glycerol, ethanol, propylene glycol, polyethylene glycol and the like into water. However, because organic solvents would easily lead to pollution, affecting the health of operators, they are not widely used.

As for adding a solubilizer to improve the drug solubility, when the addition of another small molecule substance to an aqueous solution of a drug enhances the drug solubility without reducing its physiological activity, this small molecule substance is referred to as a solubilizer. The mechanism of solubilization includes the formation of soluble complexes, complex salts, composites, etc. The common solubilizers include inorganic compounds such as potassium iodide and the like, organic acids and sodium salts thereof such as sodium benzoate, sodium salicylate and the like, as well as amines or amides such as ethylenediamine, urea, acetamide and nicotinamide. However, the above method has a slow speed for dissolving drugs, and the redissolution speed of the prepared water-soluble drug is not very ideal, with the fastest dissolution speed above 10 s, and the composition of the prepared water-soluble drug is unstable and has low drug content, limiting industrial production of the drug. Therefore, providing a solubilizer which is capable of increasing the solubility of poorly soluble drugs, and has a fast dissolution speed, safety and is suitable for industrial production, and is of practical significance.

Gastrodin, 4-hydroxymethylphenyl-β-D-glucopyranoside hemihydrate, has a relatively stable structure of phenolic glycoside, and the structure thereof is shown in formula I:

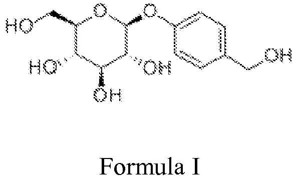

Gastrodin is a white crystalline powder, odorless, bitter taste; melting point: 153 °C~156 °C; easily soluble in water or methanol and soluble in ethanol. Gastrodin has sedative, hypnotic and analgesic effects, and has low toxicity. It can be calculated from the maximum tolerance dose of intramuscular injection and intravenous injection for animals that the maximum tolerance dose for human is 33.249 g of gastrodin (calculated at 60 kg). Gastrodin has excellent solubility in water, which may reach up to 50% or more, but research thereof as a solubilizer has not been reported yet.

### SUMMARY OF THE INVENTION

In view of this, the present invention provides the use of gastrodin as a solubilizer and a method for preparing an injection or a powder for injection. The dissolution speed of the method is fast, redissolution tests show that the powder for injection prepared by using gastrodin as the solubilizer respectively for a flavonoid compound, a carbon glycoside compound and a stilbene compound redissolves completely in only five seconds, has a significant increase in the redissolution speed as compared with a control group; also, the stability is good, which meets the requirement for clinical emergencies.

In order to achieve the above objectives, the present invention provides the following technical solutions:

The present invention provides the use of gastrodin as a solubilizer for a flavonoid compound, a carbon glycoside compound or a stilbene compound.

Preferably, the present invention provides the use of gastrodin as a solubilizer for a stilbene compound.

Preferably, the flavonoid compound is selected from the group consisting of flavone, isoflavone, flavonol, dihydroisoflavone, dihydroflavone, isoflavane, dihydroflavonol, rotenone, anthocyanin, pterocarpin, flavan-3-ol, aurone, flavan-3,4-diol, isoaurone, chalcone, homoflavone, dihydrochalcone, phenethylchromone, xanthone or homoisoflavone.

Preferably, the flavone is selected from the group consisting of baicalin, baicalein, wogonin or breviscapine; the isoflavone is selected from the group consisting of daidzein, daidzin or puerarin; the flavonol is selected from the group consisting of kaempferide or quercetin; the dihydroflavone is selected from the group consisting of dihydrobaicalein, taxinine, naringenin or hesperetin; the pterocarpin can be maackiain; and the homoisoflavone can be homoisoflavone.

The carbon glycoside compound is selected from the group consisting of barbaloin, homoorientin, mangiferin, bergenin or aloesin.

The stilbene compound is selected from the group consisting of resveratrol, piceatannol, polydatin, triptophenolide, rhapontin, deoxyrhapontin, as well as a polymer formed by one or more of resveratrol, piceatannol, polydatin, triptophenolide, rhapontin and deoxyrhapontin.

The stilbene compound refers to a general term of monomers and polymers having 1,2-diphenylethene and derivates thereof, including diphenylethylene glycosides, diphenylethyl compounds, phenanthrenes and derivatives thereof The diphenylethylene compounds mainly have antibacterial, antifungal, hypolipidemic, hepatoprotective, antioxidant, antithrombotic, coronary vessel-expanding, antihypertensive, anti-allergy, platelet aggregation-inhibiting, anti-tumor and anti-HIV and other effects, but also have effects of inhibiting activities of DNA polymerase, antagonism of estrogen and inhibiting the growth of human breast cancer.

The diphenylethylene glycoside compounds are mainly monomer derivatives or polymers (such as polydatin, triptophenolide, rhapontin and deoxyrhapontin [methyl polydatin]) with resveratrol, piceatannol or hydrides thereof as basic units in structure, which are divided into resveratrol oligomer analogues, isorhapontigenin oligomer analogues, piceatannol oligomer analogues, resveratrol and oxyresveratrol oligomers, glycosides formed by resveratrol oligomers, of which the linking sugars are all glucoses with the number varying from 1 to 3, and the forms of glycosides are oxyglycoside and glycoside. Preferably, the stilbene compound is selected from the group consisting of resveratrol, piceatannol, polydatin, triptophenolide, rhapontin, dexoyrhapontin, or a polymer formed by one or more of resveratrol, piceatannol, polydatin, triptophenolide, rhapontin and dexoyrhapontin.

Preferably, the stilbene compound is triptophenolide, polydatin, resveratrol, piceatannol, rhapontin or dexoyrhapontin.

Triptophenolide is a compound of (E)-1-(3,5-dihydroxyphenyl)-2-(3-hydroxy-4-O-β-D-glucopyranosylphenyl)ethylene, or called 3,5,3',4'-tetrahydroxystilbene-3'-O-β-glucoside (also called 3,5,4'-trihydroxy-stilbene-3'-glucoside), and the chemical structure is shown in formula II:

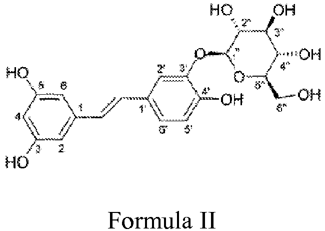

The present invention further provides a method of dissolving a flavonoid compound, a carbon glycoside compound or a stilbene compound by using gastrodin, including the following steps:

mixing the flavonoid compound, the carbon glycoside compound or the stilbene compound with gastrodin to obtain a reactant; and

heating water for injection to 80-98 °C, followed by stirring under vacuum conditions at 30-80 °C, and then adding the reactant under the protection of nitrogen gas or argon gas to obtain a solution of the reactant.

Considering the sensitivity to heat or light of the flavonoid compound, the carbon glycoside compound or the stilbene compound, the present invention provides a method by heating water for injection to 80-98 °C to remove dissolved oxygen and other gases in water, reduce the surface tension of water, increase the contact area of water with the flavonoid compound, the carbon glycoside compound or the stilbene compound, thus enhancing the dissolution speed. Meanwhile, pre-heating the water for injection can effectively avoid degradation of the flavonoid compound, the carbon glycoside compound or the stilbene compound due to exposure to the dissolved oxygen, to eliminate unstable factors of the flavonoid compound, the carbon glycoside compound or the stilbene compound. The vacuum conditions can achieve further deoxygenation, facilitating the dissolution and stabilization of the flavonoid compound, the carbon glycoside compound or the stilbene compound in water. Nitrogen gas or argon gas provides an inert atmosphere, which further reduces the surface tension of the materials, increases the contact area of the flavonoid compound, the carbon glycoside compound or the stilbene compound with water, and increases the dissolution speed.

Stirring can accelerate the removal of the dissolved oxygen in water, thus improving the dissolution speed. However, due to the unstability of the flavonoid compound, the carbon glycoside compound or the stilbene compound, stirring for too long time will easily lead to the degradation of the flavonoid compound, the carbon glycoside compound or the stilbene compound. Therefore, controlling the stirring time may increase the dissolution speed while keeping the flavonoid compound, the carbon glycoside compound or the stilbene compound stable. Preferably, the stirring time is 1~60 min.

Preferably, the mass ratio of the flavonoid compound, the carbon glycoside compound or the stilbene compound to the gastrodin is 1:0.1~80.

Preferably, the mass ratio of the flavonoid compound, the carbon glycoside compound or the stilbene compound to the gastrodin is 1:1~20.

Preferably, characterized in that the mass ratio of the flavonoid compound, the carbon glycoside compound or the stilbene compound to the gastrodin is 1:3~10.

Preferably, the mass-to-volume ratio of the gastrodin to the solution of the reactant is 2%~60% in g/mL.

Preferably, the mass-to-volume ratio of the gastrodin to the solution of the reactant is 3%~50% in g/mL.

Preferably, the mass-to-volume ratio of the gastrodin to the solution of the reactant is 3%~28% in g/mL.

The present invention provides a method for preparing an injection of a flavonoid compound, a carbon glycoside compound or a stilbene compound, including the following steps:

mixing the flavonoid compound, the carbon glycoside compound or the stilbene compound with a solubilizer to obtain a reactant;

heating water for injection to 80~98 °C, followed by stirring under vacuum conditions at 30~80 °C, and then adding the reactant under the protection of nitrogen gas or argon gas, to obtain a solution of the reactant; and adjusting pH value, filtering, filling and sterilizing to obtain the injection.

Preferably, the stirring time is 1~60 min.

The present invention provides a method for preparing a powder for injection of a flavonoid compound, a carbon glycoside compound or a stilbene compound, including the following steps:

mixing the flavonoid compound, the carbon glycoside compound or the stilbene compound with a solubilizer to obtain a reactant;

heating water for injection to 80~98 °C, followed by stirring under vacuum conditions at 30~80 °C, and then adding the reactant under the protection of nitrogen gas or argon gas, to obtain a solution of the reactant; and adjusting pH value, sterile-filtering, sterile-filling and freeze-drying to obtain the powder for injection.

Preferably, the stirring time is 1~60 min.

The present invention provides the use of gastrodin as a solubilizer and a method for preparing an injection or a powder for injection. Redissolution tests show that the powder for injection prepared by using gastrodin as the solubilizer respectively for a flavonoid compound, a carbon glycoside compound, or a stilbene compound dissolves completely in only five seconds, and has a significant increase in the redissolution speed as compared with a control group. Stability tests show that, a sample prepared from a traditional mixed solvent of PEG400 and mannitol has a degree of content degradation above 8%, failing to meet the requirements of stability for pharmaceutical preparations; while in the present invention, the freeze-dried powder for injection of water-soluble drugs prepared by using gastrodin as the solubilizer respectively for the flavonoid compound, the carbon glycoside compound and the stilbene compound with a fast dissolution speed, as compared with that before placed, has no change in properties and related substances, no significant change in pH value and content, the stability is good, which meets the medicinal requirement for clinical emergencies.

### DETAILED EMBODIMENTS

The present invention discloses the use of gastrodin as a solubilizer and a method for preparing an injection or a powder for injection, and those skilled in the art can use the content herein for reference and achieve the present invention by appropriately improving the process parameters. It should be particularly noted that all the similar replacements and modifications are obvious to those skilled in the art, which are deemed to be included in the present invention. The method and use of the present invention have been described by preferred examples, and related persons may obviously make modifications or appropriate changes or combinations to the method and use described herein without departing from the content, spirit and scope of the present invention, to achieve and apply the technique of the present invention.

Reagents used in the use of gastrodin as the solubilizer provided in the present invention are all commercially available.

The present invention will be further illustrated in conjunction with the following Examples:

### Example 1: Solubility of triptophenolide in aqueous solutions with different gastrodin concentrations

Solubility is shown in table 1.

**Table 1. Solubility of triptophenolide in aqueous solutions with different gastrodin concentrations**

| Gastrodin concentration (%) | Solubility of triptophenolide (mg/ml) |
|---|---|
| 0 | 1.752 |
| 1 | 3.191 |
| 3 | 5.097 |
| 5 | 7.107 |
| 8 | 9.169 |
| 10 | 11.438 |
| 15 | 11.761 |
| 25 | 38.378 |
| 30 | 60.424 |
| 35 | 87.793 |
| 40 | 85.117 |
| 45 | 97.993 |
| 50 | 105.017 |

It can be seen from data in table 1 that gastrodin can improve the solubility of triptophenolide in water.

### Example 2: Preparation of a freeze-dried powder for injection by dissolving a flavonoid compound

| Formula: | |
|---|---|
| Breviscapine | 5 g |
| Gastrodin | 100 g |
| Sodium carbonate | 10 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing breviscapine along with gastrodin and sodium carbonate weighed according to the amounts in the formula by nitrogen gas into the preparation tank. Breviscapine dissolved in about 5 minutes, then vacuum and nitrogen gas were removed, followed by adjusting pH and content and adding water to the total amount, thus completing the preparation process. Next, it is sterile-filtered, sterile-filled and freeze-dried by a conventional process to obtain the freeze-dried powder for injection.

### Example 3: Preparation of a freeze-dried powder for injection by dissolving a flavonoid compound

| Formula: | |
|---|---|
| Breviscapine | 5 g |
| Gastrodin | 100 g |
| Sodium bisulfite | 0.2 g |
| Disodium edetate | 0.1 g |
| Sodium carbonate | 5 g |
| Water for injection | 1000 ml |

60% of the total amount of water for injection for preparation was added into a preparation tank, heated to 90 °C with slight boiling, and kept at 80 °C. The vacuum was initiated while stirring for 60 minutes, followed by bringing breviscapine along with gastrodin, disodium edetate and sodium carbonate weighed according to the amounts in the formula by nitrogen gas into the preparation tank. Breviscapine dissolved in about 5 minutes, then vacuum and nitrogen gas were removed, sodium bisulfite was added in a conventional way, followed by adjusting pH and content and adding water to the total amount, thus completing the preparation process. Next, it is sterile-filtered, sterile-filled and freeze-dried by a conventional process to obtain the freeze-dried powder for injection.

### Example 4: Preparation of a freeze-dried powder for injection by dissolving a flavonoid compound

| Formula: | |
|---|---|
| Breviscapine | 5 g |
| Gastrodin | 100 g |
| Sodium sulfite | 0.2 g |
| Disodium edetate | 0.1 g |
| Sodium carbonate | 5 g |
| Water for injection | 1000 ml |

60% of the total amount of water for injection for preparation was added into a preparation tank, heated to 98 °C with slight boiling, and kept at 80 °C. The vacuum was initiated while stirring for 20 minutes, followed by bringing breviscapine along with gastrodin, disodium edetate and sodium carbonate weighed according to the amounts in the formula by nitrogen gas into the preparation tank. Breviscapine dissolved in about 5 minutes, then vacuum and nitrogen gas were removed, sodium sulfite was added in a conventional way, followed by adjusting pH and content and adding water to the total amount, thus completing the preparation process. Next, it is sterile-filtered, sterile-filled and freeze-dried by a conventional process to obtain the freeze-dried powder for injection.

### Example 5: Preparation of a freeze-dried powder for injection by dissolving a flavonoid compound

| Formula: | |
|---|---|
| Breviscapine | 5 g |
| Gastrodin | 100 g |
| Vitamin C | 0.2 g |
| Disodium edetate | 0.1 g |
| Sodium carbonate | 5 g |
| Water for injection | 1000 ml |

60% of the total amount of water for injection for preparation was added into a preparation tank, heated to 85 °C with slight boiling, and kept at 80 °C. The vacuum was initiated while stirring for 30 minutes, followed by bringing breviscapine along with gastrodin, disodium edetate and sodium carbonate weighed according to the amounts in the formula by nitrogen gas into the preparation tank. Breviscapine dissolved in about 5 minutes, then vacuum and nitrogen gas were removed, vitamin C was added in a conventional way, followed by adjusting pH and content and adding water to the total amount, thus completing the preparation process. Next, it is sterile-filtered, sterile-filled and freeze-dried by a conventional process to obtain the freeze-dried powder for injection.

### Example 6: Preparation of a freeze-dried powder for injection by dissolving a flavonoid compound

| Formula: | |
|---|---|
| Breviscapine | 5 g |
| Gastrodin | 100 g |
| L-cysteine hydrochloride | 0.2 g |
| Disodium edetate | 0.1 g |

| | |
|---|---|
| Sodium carbonate | 5 g |
| Water for injection | 1000 ml |

60% of the total amount of water for injection for preparation was added into a preparation tank, heated to 95 °C with slight boiling, and kept at 80 °C. The vacuum was initiated while stirring for 40 minutes, followed by bringing breviscapine along with gastrodin, disodium edetate and sodium carbonate weighed according to the amounts in the formula by nitrogen gas into the preparation tank. Breviscapine dissolved in about 5 minutes, then vacuum and nitrogen gas were removed, L-cysteine hydrochloride was added in a conventional way, followed by adjusting pH and content and adding water to the total amount, thus completing the preparation process. Next, it is sterile-filtered, sterile-filled and freeze-dried by a conventional process to obtain the freeze-dried powder for injection.

### Example 7: Preparation of a freeze-dried powder for injection by dissolving a flavonoid compound

| Formula: | |
|---|---|
| Breviscapine | 5 g |
| Gastrodin | 100 g |
| Glutathione | 0.2 g |
| Disodium edetate | 0.1 g |
| Sodium carbonate | 5 g |
| Water for injection | 1000 ml |

60% of the total amount of water for injection for preparation was added into a preparation tank, heated to 98 °C with slight boiling, and kept at 80 °C. The vacuum was initiated while stirring for 50 minutes, followed by bringing breviscapine along with gastrodin, disodium edetate and sodium carbonate weighed according to the amounts in the formula by nitrogen gas into the preparation tank. Breviscapine dissolved in about 5 minutes, then vacuum and nitrogen gas were removed, glutathione was added in a conventional way, followed by adjusting pH and content and adding water to the total amount, thus completing the preparation process. Next, it is sterile-filtered, sterile-filled and freeze-dried by a conventional process to obtain the freeze-dried powder for injection.

### Example 8: Preparation of a freeze-dried powder for injection by dissolving a flavonoid compound

| | |
|---|---|
| Formula: | |
| Breviscapine | 5 g |
| Gastrodin | 100 g |
| Glutathione sodium | 0.2 g |
| Disodium edetate | 0.1 g |
| Sodium carbonate | 2 g |
| Water for injection | 1000 ml |

60% of the total amount of water for injection for preparation was added into a preparation tank, heated to 96 °C with slight boiling, and kept at 80 °C. The vacuum was initiated while stirring for 30 minutes, followed by bringing breviscapine along with gastrodin, disodium edetate and sodium carbonate weighed according to the amounts in the formula by nitrogen gas into the preparation tank. Breviscapine dissolved in about 5 minutes, then vacuum and nitrogen gas were removed, glutathione sodium was added in a conventional way, followed by adjusting pH and content and adding water to the total amount, thus completing the preparation process. Next, it is sterile-filtered, sterile-filled and freeze-dried by a conventional process to obtain the freeze-dried powder for injection.

### Example 9: Preparation of a freeze-dried powder for injection by dissolving a flavonoid compound

| | |
|---|---|
| Formula: | |
| Baicalin | 5 g |
| Gastrodin | 100 g |
| Sodium carbonate | 10 g |

| | |
|---|---|
| Water for injection | 1000 ml |

60% of the total amount of water for injection for preparation was added into a preparation tank, heated to 94 °C with slight boiling, and kept at 80 °C. The vacuum was initiated while stirring for 50 minutes, followed by bringing baicalin along with gastrodin and sodium carbonate weighed according to the amounts in the formula by nitrogen gas into the preparation tank. Baicalin dissolved in about 5 minutes, then vacuum and nitrogen gas were removed, followed by adjusting pH and content and adding water to the total amount, thus completing the preparation process. Next, it is sterile-filtered, sterile-filled and freeze-dried by a conventional process to obtain the injection.

### Example 10: Preparation of a freeze-dried powder for injection by dissolving a flavonoid compound

| Formula: | |
|---|---|
| Baicalein | 5 g |
| Gastrodin | 100 g |
| Sodium bisulfite | 0.2 g |
| Disodium edetate | 0.1 g |
| Sodium carbonate | 5 g |
| Water for injection | 1000 ml |

60% of the total amount of water for injection for preparation was added into a preparation tank, heated to 97 °C with slight boiling, and kept at 80 °C. The vacuum was initiated while stirring for 20 minutes, followed by bringing baicalein along with gastrodin, disodium edetate and sodium carbonate weighed according to the amounts in the formula by nitrogen gas into the preparation tank. Baicalein dissolved in about 5 minutes, then vacuum and nitrogen gas were removed, sodium bisulfite was added in a conventional way, followed by adjusting pH and content and adding water to the total amount, thus completing the preparation process. Next, it is sterile-filtered, sterile-filled and freeze-dried by a conventional process to obtain the injection.

### Example 11: Preparation of an injection by dissolving a flavonoid compound

| Formula: | |
|---|---|
| Wogonin | 5 g |
| Gastrodin | 100 g |
| Sodium sulfite | 0.2 g |
| Disodium edetate | 0.1 g |
| Sodium carbonate | 5 g |
| Water for injection | 1000 ml |

60% of the total amount of water for injection for preparation was added into a preparation tank, heated to 95 °C with slight boiling, and kept at 80 °C. The vacuum was initiated while stirring for 60 minutes, followed by bringing wogonin along with gastrodin, disodium edetate and sodium carbonate weighed according to the amounts in the formula by nitrogen gas into the preparation tank. Wogonin dissolved in about 5 minutes, then vacuum and nitrogen gas were removed, sodium sulfite was added in a conventional way, followed by adjusting pH and content and adding water to the total amount, thus completing the preparation process. Next, it is filtered, filled and sterilized by a conventional process to obtain the injection.

### Example 12: Preparation of an injection by dissolving a flavonoid compound

| Formula: | |
|---|---|
| Daidzein | 5 g |
| Gastrodin | 100 g |
| Vitamin C | 0.2 g |
| Disodium edetate | 0.1 g |
| Sodium carbonate | 3 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing daidzein along with gastrodin, disodium edetate and sodium carbonate weighed according to the amounts in the formula by nitrogen gas into the preparation tank. Daidzein dissolved in about 5 minutes, then vacuum and nitrogen gas were removed, vitamin C was added in a conventional way, followed by adjusting pH and content and adding water to the total amount, thus completing the preparation process. Next, it is filtered, filled and sterilized by a conventional process to obtain the injection.

### Example 13: Preparation of an injection by dissolving a flavonoid compound

| Formula: | |
|---|---|
| Daidzin | 5 g |
| Gastrodin | 100 g |
| L-cysteine hydrochloride | 0.2 g |
| Disodium edetate | 0.1 g |
| Sodium carbonate | 5 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing daidzin along with gastrodin, disodium edetate and sodium carbonate weighed according to the amounts in the formula by nitrogen gas into the preparation tank. Daidzin dissolved in about 5 minutes, then vacuum and nitrogen gas were removed, L-cysteine hydrochloride was added in a conventional way, followed by adjusting pH and content and adding water to the total amount, thus completing the preparation process. Next, it is filtered, filled and sterilized by a conventional process to obtain the injection.

### Example 14: Preparation of an injection by dissolving a flavonoid compound

| Formula: | |
|---|---|
| Puerarin | 5 g |
| Gastrodin | 100 g |
| Glutathione | 0.2 g |
| Disodium edetate | 0.1 g |
| Sodium carbonate | 5 g |
| Water for injection | 1000 ml |

60% of the total amount of water for injection for preparation was added into a preparation tank, heated to 98 °C with slight boiling, and kept at 80 °C. The vacuum was initiated while stirring for 10 minutes, followed by bringing puerarin along with gastrodin, disodium edetate and sodium carbonate weighed according to the amounts in the formula by nitrogen gas into the preparation tank. Puerarin dissolved in about 5 minutes, then vacuum and nitrogen gas were removed, glutathione was added in a conventional way, followed by adjusting pH and content and adding water to the total amount, thus completing the preparation process. Next, it is filtered, filled and sterilized by a conventional process to obtain the injection.

### Example 15: Preparation of an injection by dissolving a flavonoid compound

| Formula: | |
|---|---|
| Kaempferide | 5 g |
| Gastrodin | 100 g |
| Glutathione sodium | 0.2 g |
| Disodium edetate | 0.1 g |
| Sodium carbonate | 2 g |
| Water for injection | 1000 ml |

60% of the total amount of water for injection for preparation was added into a preparation tank, heated to 96 °C with slight boiling, and kept at 80 °C. The vacuum was initiated while stirring for 40 minutes, followed by bringing kaempferide along with gastrodin, disodium edetate and sodium carbonate weighed according to the amounts in the formula by nitrogen gas into the preparation tank. Kaempferide dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 16: Preparation of an injection by dissolving a flavonoid compound

| Formula: | |
|---|---|
| Quercetin | 5 g |
| Gastrodin | 100 g |
| Glutathione | 0.2 g |
| Disodium edetate | 0.1 g |
| Sodium carbonate | 4 g |
| Water for injection | 1000 ml |

60% of the total amount of water for injection for preparation was added into a preparation tank, heated to 95 °C with slight boiling, and kept at 80 °C. The vacuum was initiated while stirring for 20 minutes, followed by bringing quercetin along with gastrodin, disodium edetate and sodium carbonate weighed according to the amounts in the formula by nitrogen gas into the preparation tank. Quercetin dissolved in about 5 minutes, then vacuum and nitrogen gas were removed, glutathione was added in a conventional way, followed by adjusting pH and content and adding water to the total amount, thus completing the preparation process. Next, it is filtered, filled and sterilized by a conventional process to obtain the injection.

### Example 17: Preparation of an injection by dissolving a flavonoid compound

| Formula: | |
|---|---|
| Dihydrobaicalein | 5 g |
| Gastrodin | 100 g |
| Glutathione sodium | 0.2 g |
| Disodium edetate | 0.1 g |
| Sodium carbonate | 4 g |
| Water for injection | 1000 ml |

60% of the total amount of water for injection for preparation was added into a preparation tank, heated to 92 °C with slight boiling, and kept at 80 °C. The vacuum was initiated while stirring for 50 minutes, followed by bringing dihydrobaicalein along with gastrodin, disodium edetate and sodium carbonate weighed according to the amounts in the formula by nitrogen gas into the preparation tank. Dihydrobaicalein dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 18: Preparation of an injection by dissolving a flavonoid compound

| Formula: | |
|---|---|
| Taxinine | 5 g |
| Gastrodin | 100 g |
| Glutathione sodium | 0.2 g |
| Disodium edetate | 0.1 g |
| Sodium carbonate | 4 g |
| Water for injection | 1000 ml |

60% of the total amount of water for injection for preparation was added into a preparation tank, heated to 91 °C with slight boiling, and kept at 80 °C. The vacuum was initiated while stirring for 55 minutes, followed by bringing taxinine along with gastrodin, disodium edetate and sodium carbonate weighed according to the amounts in the formula by nitrogen gas into the preparation tank. Taxinine dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 19: Preparation of an injection by dissolving a flavonoid compound

| Formula: | |
|---|---|
| Naringenin | 5 g |
| Gastrodin | 100 g |
| Glutathione sodium | 0.2 g |
| Disodium edetate | 0.1 g |
| Sodium carbonate | 3 g |
| Water for injection | 1000 ml |

60% of the total amount of water for injection for preparation was added into a preparation tank, heated to 87 °C with slight boiling, and kept at 80 °C. The vacuum was initiated while stirring for 60 minutes, followed by bringing naringenin along with gastrodin, disodium edetate and sodium carbonate weighed according to the amounts in the formula by nitrogen gas into the preparation tank. Naringenin dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 20: Preparation of an injection by dissolving a flavonoid compound

| Formula: | |
|---|---|
| Hesperetin | 5 g |

| | |
|---|---|
| Gastrodin | 100 g |
| Glutathione sodium | 0.2 g |
| Disodium edetate | 0.1 g |
| Sodium carbonate | 3 g |
| Water for injection | 1000 ml |

60% of the total amount of water for injection for preparation was added into a preparation tank, heated to 85 °C with slight boiling, and kept at 80 °C. The vacuum was initiated while stirring for 40 minutes, followed by bringing hesperetin along with gastrodin, disodium edetate and sodium carbonate weighed according to the amounts in the formula by nitrogen gas into the preparation tank. Hesperetin dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 21: Preparation of an injection by dissolving a flavonoid compound

| Formula: | |
|---|---|
| Maackiain | 5 g |
| Gastrodin | 100 g |
| Glutathione sodium | 0.2 g |
| Disodium edetate | 0.1 g |
| Sodium carbonate | 3 g |
| Water for injection | 1000 ml |

60% of the total amount of water for injection for preparation was added into a preparation tank, heated to 80 °C with slight boiling, and kept at 80 °C. The vacuum was initiated while stirring for 60 minutes, followed by bringing maackiain along with gastrodin, disodium edetate and sodium carbonate weighed according to the amounts in the formula by nitrogen gas into the preparation tank. Maackiain dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 22: Preparation of an injection by dissolving a flavonoid compound

| Formula: | |
|---|---|
| Homoisoflavone | 5 g |
| Gastrodin | 100 g |
| Glutathione sodium | 0.2 g |
| Disodium edetate | 0.1 g |
| Sodium carbonate | 3 g |
| Water for injection | 1000 ml |

60% of the total amount of water for injection for preparation was added into a preparation tank, heated to 88 °C with slight boiling, and kept at 80 °C. The vacuum was initiated while stirring for 25 minutes, followed by bringing homoisoflavone along with gastrodin, disodium edetate and sodium carbonate weighed according to the amounts in the formula by nitrogen gas into the preparation tank. Homoisoflavone dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 23: Preparation of a freeze-dried powder for injection by dissolving a carbon glycoside compound

| Formula: | |
|---|---|
| Mangiferin | 10 g |
| Gastrodin | 100 g |
| Sodium bicarbonate | 2 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing mangiferin along with gastrodin and sodium bicarbonate weighed according to the amounts in the formula by nitrogen gas into the preparation tank. Mangiferin dissolved in about 10 minutes, then vacuum and nitrogen gas were removed, glutathione sodium was added in a conventional way, followed by adjusting pH and content and adding water to the total amount, thus completing the preparation process. Next, it is sterile-filtered, sterile-filled and freeze-dried by a conventional process to obtain the injection.

### Example 24: Preparation of a freeze-dried powder for injection by dissolving a carbon glycoside compound

| Formula: | |
|---|---|
| Mangiferin | 10 g |
| Gastrodin | 100 g |
| Sodium bicarbonate | 2 g |
| Water for injection | 1000 ml |

40% of the total amount of water for injection for preparation was added into a preparation tank, heated to 88 °C with slight boiling, and kept at 80 °C. The vacuum was initiated while stirring for 25 minutes, followed by bringing mangiferin along with gastrodin and sodium bicarbonate weighed according to the amounts in the formula by nitrogen gas into the preparation tank. Mangiferin dissolved in about 10 minutes, then vacuum and nitrogen gas were removed, glutathione sodium was added in a conventional way, followed by adjusting pH and content and adding water to the total amount, thus completing the preparation process. Next, it is sterile-filtered, sterile-filled and freeze-dried by a conventional process to obtain the injection.

### Example 25: Preparation of a freeze-dried powder for injection by dissolving a carbon glycoside compound

| Formula: | |
|---|---|
| Mangiferin | 10 g |
| Gastrodin | 100 g |
| Sodium bicarbonate | 2 g |
| Water for injection | 1000 ml |

40% of the total amount of water for injection for preparation was added into a preparation tank, heated to 80 °C with slight boiling, and kept at 80 °C. The vacuum was initiated while stirring for 60 minutes, followed by bringing mangiferin along with gastrodin and sodium bicarbonate weighed according to the amounts in the formula by nitrogen gas into the preparation tank. Mangiferin dissolved in about 10 minutes, then vacuum and nitrogen gas were removed, glutathione sodium was added in a conventional way, followed by adjusting pH and content and adding water to the total amount, thus completing the preparation process. Next, it is sterile-filtered, sterile-filled and freeze-dried by a conventional process to obtain the injection.

### Example 26: Preparation of a freeze-dried powder for injection by dissolving a carbon glycoside compound

| Formula: | |
|---|---|
| Mangiferin | 10 g |
| Gastrodin | 100 g |
| Sodium bicarbonate | 2 g |
| Water for injection | 1000 ml |

40% of the total amount of water for injection for preparation was added into a preparation tank, heated to 98 °C with slight boiling, and kept at 80 °C. The vacuum was initiated while stirring for 1 minute, followed by bringing mangiferin along with gastrodin and sodium bicarbonate weighed according to the amounts in the formula by nitrogen gas into the preparation tank. Mangiferin dissolved in about 8 minutes, then vacuum and nitrogen gas were removed, glutathione sodium was added in a conventional way, followed by adjusting pH and content and adding water to the total amount, thus completing the preparation process. Next, it is sterile-filtered, sterile-filled and freeze-dried by a conventional process to obtain the injection.

### Example 27: Preparation of an injection by dissolving a carbon glycoside compound

| Formula: | |
|---|---|
| Bergenin | 5 g |
| Gastrodin | 100 g |
| Sodium bisulfite | 0.2 g |
| Disodium edetate | 0.1 g |
| Sodium carbonate | 1 g |
| Water for injection | 1000 ml |

60% of the total amount of water for injection for preparation was added into a preparation tank, heated to 90 °C with slight boiling, and kept at 80 °C. The vacuum was initiated while stirring for 35 minutes, followed by bringing bergenin along with gastrodin, disodium edetate and sodium carbonate weighed according to the amounts in the formula by nitrogen gas into the preparation tank. Bergenin dissolved in about 5 minutes, then vacuum and nitrogen gas were removed, sodium bisulfite was added in a conventional way, followed by adjusting pH and content and adding water to the total amount, thus completing the preparation process. Next, it is filtered, filled and sterilized by a conventional process to obtain the injection.

### Example 28: Preparation of an injection by dissolving a carbon glycoside compound

| Formula: | |
|---|---|
| Barbaloin | 5 g |
| Gastrodin | 100 g |
| Sodium bisulfite | 0.2 g |
| Disodium edetate | 0.1 g |
| Sodium carbonate | 1 g |
| Water for injection | 1000 ml |

60% of the total amount of water for injection for preparation was added into a preparation tank, heated to 95 °C with slight boiling, and kept at 80 °C. The vacuum was initiated while stirring for 20 minutes, followed by bringing barbaloin along with gastrodin, disodium edetate and sodium carbonate weighed according to the amounts in the formula by nitrogen gas into the preparation tank. Barbaloin dissolved in about 5 minutes, then vacuum and nitrogen gas were removed, sodium bisulfite was added in a conventional way, followed by adjusting pH and content and adding water to the total amount, thus completing the preparation process. Next, it is filtered, filled and sterilized by a conventional process to obtain the injection.

### Example 29: Preparation of an injection by dissolving a carbon glycoside compound

| Formula: | |
|---|---|
| Homoorientin | 5 g |
| Gastrodin | 100 g |
| Vitamin C | 0.2 g |
| Disodium edetate | 0.1 g |
| Sodium carbonate | 2 g |
| Water for injection | 1000 ml |

60% of the total amount of water for injection for preparation was added into a preparation tank, heated to 89 °C with slight boiling, and kept at 80 °C. The vacuum was initiated while stirring for 30 minutes, followed by bringing homoorientin along with gastrodin, disodium edetate and sodium carbonate weighed according to the amounts in the formula by nitrogen gas into the preparation tank. Homoorientin dissolved in about 5 minutes, then vacuum and nitrogen gas were removed, vitamin C was added in a conventional way, followed by adjusting pH and content and adding water to the total amount, thus completing the preparation process. Next, it is filtered, filled and sterilized by a conventional process to obtain the injection.

### Example 30: Preparation of an injection by dissolving a carbon glycoside compound

| Formula: | |
|---|---|
| Mangiferin | 5 g |
| Gastrodin | 100 g |
| L-cysteine hydrochloride | 0.2 g |
| Disodium edetate | 0.1 g |
| Sodium carbonate | 2 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing mangiferin along with gastrodin, disodium edetate and sodium carbonate weighed according to the amounts in the formula by nitrogen gas into the preparation tank. Mangiferin dissolved in about 5 minutes, then vacuum and nitrogen gas were removed, L-cysteine hydrochloride was added in a conventional way, followed by adjusting pH and content and adding water to the total amount, thus completing the preparation process. Next, it is filtered, filled and sterilized by a conventional process to obtain the injection.

### Example 31: Preparation of an injection by dissolving a carbon glycoside compound

| Formula: | |
|---|---|
| Aloesin | 5 g |
| Gastrodin | 100 g |
| Glutathione | 0.2 g |
| Disodium edetate | 0.1 g |
| Sodium carbonate | 2 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing aloesin along with gastrodin, disodium edetate and sodium carbonate weighed according to the amounts in the formula by nitrogen gas into the preparation tank. Aloesin dissolved in about 5 minutes, then vacuum and nitrogen gas were removed, glutathione was added in a conventional way, followed by adjusting pH and content and adding water to the total amount, thus completing the preparation process. Next, it is filtered, filled and sterilized by a conventional process to obtain the injection.

### Example 32: Preparation of an injection by dissolving a carbon glycoside compound

| Formula: | |
|---|---|
| Bergenin | 5 g |
| Gastrodin | 100 g |
| Glutathione sodium | 0.2 g |
| Disodium edetate | 0.1 g |
| Sodium carbonate | 1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing bergenin along with gastrodin, disodium edetate and sodium carbonate weighed according to the amounts in the formula by nitrogen gas into the preparation tank. Bergenin dissolved in about 5 minutes, then vacuum and nitrogen gas were removed, glutathione sodium was added in a conventional way, followed by adjusting pH and content and adding water to the total amount, thus completing the preparation process. Next, it is filtered, filled and sterilized by a conventional process to obtain the injection.

### Example 33: Preparation of a freeze-dried powder for injection by dissolving a stilbene compound

| Formula: | |
|---|---|
| Triptophenolide | 5 g |
| Gastrodin | 80 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 34: Preparation of a freeze-dried powder for injection by dissolving a stilbene compound

| Formula: | |
|---|---|
| Triptophenolide | 20 g |
| Gastrodin | 200 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 35: Preparation of a freeze-dried powder for injection by dissolving a stilbene compound

| Formula: | |
|---|---|
| Triptophenolide | 10 g |
| Gastrodin | 80 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 36: Preparation of a freeze-dried powder for injection by dissolving a stilbene compound

| Formula: | |
|---|---|
| Triptophenolide | 5 g |
| Gastrodin | 100 g |
| Sodium bisulfite | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, sodium bisulfite and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 37: Preparation of a freeze-dried powder for injection by dissolving a stilbene compound

| Formula: | |
|---|---|
| Triptophenolide | 5 g |
| Gastrodin | 100 g |
| Sodium sulfite | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, sodium sulfite and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 38: Preparation of a freeze-dried powder for injection by dissolving a stilbene compound

| Formula: | |
|---|---|
| triptophenolide | 5 g |
| Gastrodin | 100 g |
| Vitamin C | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, vitamin C and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 39: Preparation of a freeze-dried powder for injection by dissolving a stilbene compound

| Formula: | |
|---|---|
| Triptophenolide | 5 g |
| Gastrodin | 100 g |
| L-cysteine hydrochloride | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, L-cysteine hydrochloride and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 40: Preparation of a freeze-dried powder for injection by dissolving a stilbene compound

| Formula: | |
|---|---|
| Triptophenolide | 5 g |
| Gastrodin | 100 g |
| Glutathione | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, glutathione and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 41: Preparation of a freeze-dried powder for injection by dissolving a stilbene compound

| Formula: | |
|---|---|
| Triptophenolide | 5 g |
| Gastrodin | 100 g |
| Glutathione sodium | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, glutathione sodium and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 42: Preparation of a freeze-dried powder for injection by dissolving a stilbene compound

| Formula: | |
|---|---|
| Piceatannol | 5 g |
| Gastrodin | 80 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing piceatannol along with gastrodin weighed according to the amounts in the formula by nitrogen gas into the preparation tank. Piceatannol dissolved in about 5 minutes, then vacuum and nitrogen gas were removed, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 43: Preparation of a freeze-dried powder for injection by dissolving a stilbene compound

| Formula: | |
|---|---|
| Resveratrol | 20 g |
| Gastrodin | 200 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing resveratrol along with gastrodin weighed according to the amounts in the formula by nitrogen gas into the preparation tank. Resveratrol dissolved in about 5 minutes, then vacuum and nitrogen gas were removed, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 44: Preparation of a freeze-dried powder for injection by dissolving a stilbene compound

| Formula: | |
|---|---|
| Polydatin | 10 g |
| Gastrodin | 80 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing polydatin along with gastrodin weighed according to the amounts in the formula by nitrogen gas into the preparation tank. Polydatin dissolved in about 5 minutes, then vacuum and nitrogen gas were removed, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 45: Preparation of a freeze-dried powder for injection by dissolving a stilbene compound

| Formula: | |
|---|---|
| Rhapontin | 5 g |
| Gastrodin | 100 g |
| Sodium bisulfite | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, sodium bisulfite and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 46: Preparation of a freeze-dried powder for injection by dissolving a stilbene compound

| Formula: | |
|---|---|
| Deoxyrhapontin | 5 g |
| Gastrodin | 100 g |
| Sodium sulfite | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing deoxyrhapontin along with gastrodin weighed according to the amounts in the formula by nitrogen gas into the preparation tank. Deoxyrhapontin dissolved in about 5 minutes, then vacuum and nitrogen gas were removed, sodium sulfite and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 47: Preparation of a freeze-dried powder for injection by dissolving a stilbene compound

| Formula: | |
|---|---|
| Rhapontin | 5 g |
| Gastrodin | 100 g |
| Vitamin C | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing rhapontin along with gastrodin weighed according to the amounts in the formula by nitrogen gas into the preparation tank. Rhapontin dissolved in about 5 minutes, then vacuum and nitrogen gas were removed, vitamin C and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 48: Preparation of a freeze-dried powder for injection by dissolving a stilbene compound

| Formula: | |
|---|---|
| Piceatannol | 5 g |
| Gastrodin | 100 g |
| L-cysteine hydrochloride | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing piceatannol along with gastrodin weighed according to the amounts in the formula by nitrogen gas into the preparation tank. Piceatannol dissolved in about 5 minutes, then vacuum and nitrogen gas were removed, L-cysteine hydrochloride and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 49: Preparation of a freeze-dried powder for injection by dissolving a stilbene compound

| Formula: | |
|---|---|
| Resveratrol | 5 g |
| Gastrodin | 100 g |
| Glutathione | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing resveratrol along with gastrodin weighed according to the amounts in the formula by nitrogen gas into the preparation tank. Resveratrol dissolved in about 5 minutes, then vacuum and nitrogen gas were removed, glutathione and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 50: Preparation of a freeze-dried powder for injection by dissolving a stilbene compound

| Formula: | |
|---|---|
| Polydatin | 5 g |
| Gastrodin | 100 g |
| Glutathione sodium | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing polydatin along with gastrodin weighed according to the amounts in the formula by nitrogen gas into the preparation tank. Polydatin dissolved in about 5 minutes, then vacuum and nitrogen gas were removed, glutathione sodium and disodium edetate were added, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 51: Preparation of a freeze-dried powder for injection by dissolving a stilbene compound

| Formula: | |
|---|---|
| Triptophenolide | 5 g |
| Gastrodin | 0.5 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, followed by adjusting pH and content by using sodium hydroxide or hydrochloric acid, and adding water to the total amount, thus completing the preparation process. Next, it is sterile-filtered, sterile-filled and freeze-dried by a conventional process to obtain the freeze-dried powder for injection.

### Example 52: Redissolution tests on freeze-dried powder for injections prepared by dissolving flavonoid compounds

2 ml of water for injection was injected into the freeze-dried powder for injection prepared in Example 2 to Example 10 respectively, and redissolution was investigated over time without shaking. Test results were shown in Table 1.

**Table 1 Results of Redissolution speed tests on freeze-dried powder for injections prepared by dissolving flavonoid compounds**

| Groups | | Dissolving time (s) |
|---|---|---|
| | Example 2 | 4 |
| | Example 3 | 4 |
| | Example 4 | 5 |
| | Example 5 | 5 |
| Test group | Example 6 | 4 |
| | Example 7 | 5 |
| | Example 8 | 4 |
| | Example 9 | 4 |
| | Example 10 | 5 |
| Control group | A sample prepared in a mixed solvent of PEG400 and mannitol | 32 |

The redissolution tests showed that the freeze-dried powder of the flavonoid compound prepared by freeze-drying the water-soluble drug using gastrodin as the solubilizer dissolved completely in only 5 seconds, and had a significant increase in the redissolution speed as compared with the control group, which meets the requirement for clinical emergencies.

### Example 53: Redissolution speed tests on freeze-dried powder for injections prepared by dissolving carbon glycoside compounds

2 ml of water for injection was injected into the freeze-dried powder for injection prepared in Example 23 to Example 26 respectively, and redissolution was investigated over time without shaking. Test results were shown in Table 2.

**Table 2 Results of Redissolution speed tests on freeze-dried powder prepared by dissolving carbon glycoside compounds**

| Groups | | Dissolving time(s) |
|---|---|---|
| Test group | Example 23 | 2 |
| | Example 24 | 2 |
| | Example 25 | 2 |
| | Example 26 | 2 |
| Control group | A sample prepared in a mixed solvent of PEG400 and mannitol | 30 |

The redissolution tests showed that the freeze-dried powder of the carbon glycoside compound prepared by freeze-drying the water-soluble drug using gastrodin as the solubilizer dissolved completely in only 2 seconds, and had a significant increase in the redissolution speed as compared with the control group, which meets the requirement for clinical emergencies.

### Example 54: Redissolution tests on freeze-dried powder prepared by dissolving stilbene compounds

2 ml of water for injection was injected into the freeze-dried powder for injection prepared in Example 33 to Example 51 respectively, and redissolution was investigated over time without shaking. Test results were shown in Table 3.

**Table 3 Results of Redissolution speed tests on freeze-dried powder prepared by dissolving stilbene compounds**

| Groups | | Dissolving time (s) |
|---|---|---|
| | Example 33 | 2 |
| | Example 34 | 2 |
| Test group | Example 35 | 2 |
| | Example 36 | 2 |
| | Example 37 | 3 |
| | Example 38 | 3 |
| | Example 39 | 2 |
| | Example 40 | 3 |
| | Example 41 | 2 |
| | Example 42 | 2 |
| | Example 43 | 2 |
| | Example 44 | 2 |
| | Example 45 | 2 |
| | Example 46 | 3 |
| | Example 47 | 2 |
| | Example 48 | 3 |
| | Example 49 | 2 |
| | Example 50 | 2 |
| | Example 51 | 3 |
| Control group | A sample prepared in a mixed solvent of PEG400 and mannitol | 26 |

The redissolution tests showed that the freeze-dried powder of the stilbene compound prepared by freeze-drying the water-soluble drug using gastrodin as the solubilizer dissolved completely in only 3 seconds, and had a significant increase in the redissolution speed as compared with the control group, which meets the requirement for clinical emergencies.

### Example 55: Stability tests

Stability tests were performed on the freeze-dried powder prepared in the present invention.

High-temperature test: the injections of the flavonoid compounds prepared in Example 2 to Example 22, the injections of the carbon glycoside compounds prepared in Example 23 to Example 32, and the injections of the stilbene compounds prepared in Example 33 to Example 51 were placed into an incubator of 60 °C respectively, and taken out to be detected according to the investigation items on day 5 and 10. Test results were shown in Table 4 to Table 6.

**Table 4 High-temperature test results of injections of the flavonoid compounds**

| Group | | Conditions | Properties | Related substances (total area of related substances) | pH value | Content (%) |
|---|---|---|---|---|---|---|
| Control group | A sample prepared in a mixed solvent of PEG400 and mannitol | Before placed | Light yellow | Less than the main peak area of control solution | 6.21 | 99.8 |
| | | 60 °C, 5 days | Light yellow | Less than the main peak area of control solution | 5.21 | 94.48 |
| | | 60 °C, 10 days | Light yellow | Less than the main peak area of control solution | 4.72 | 88.65 |
| Test group | Freeze-dried powder of water-soluble flavonoid | Before placed | Light yellow | Less than the main peak area of control solution | 6.60 | 100.65 |
| | | 60 °C, 5 days | Light yellow | Less than the main peak | 6.44 | 100.01 |
| | compounds | | | area of control solution | | |
| | | 60 °C, 10 days | Light yellow | Less than the main peak area of control solution | 6.30 | 98.65 |

**Table 5 High-temperature test results of injections of the carbon glycoside compounds**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Light yellow | Less than the main peak area of control solution | 6.05 | 99.5 |
| | | 60 °C, 5 days | Light yellow | Less than the main peak area of control solution | 5.05 | 93.01 |
| | | 60 °C, 10 days | Light yellow | Less than the main peak area of control solution | 4.08 | 86.72 |
| Test group | Freeze-dried powder of water-soluble | Before placed | Light yellow | Less than the main peak area of | 6.55 | 100.07 |
| | carbon glycoside compounds | | | control solution | | |
| | | 60 °C, 5 days | Light yellow | Less than the main peak area of control solution | 6.32 | 99.78 |
| | | 60 °C, 10 days | Light yellow | Less than the main peak area of control solution | 6.24 | 97.96 |

**Table 6 High-temperature test results of injections of the stilbene compounds**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Off-white | Less than the main peak area of control solution | 4.07 | 94.13 |
| | | 60 °C, 5 days | Light red | Less than the main peak area of control solution | 4.15 | 83.01 |
| | | 60 °C, 10 days | Light red | Less than the main peak area of control | 3.98 | 74.51 |
| | | | | solution | | |
| Test group | Freeze-dried powder for injection of water-soluble stilbene compounds | Before placed | White | Less than the main peak area of control solution | 5.41 | 100.77 |
| | | 60 °C, 5 days | White | Less than the main peak area of control solution | 5.09 | 99.87 |
| | | 60 °C, 10 days | White | Less than the main peak area of control solution | 5.01 | 95.88 |

Strong light exposure tests: the injections of the flavonoid compounds prepared in Example 2 to Example 22, the injections of the carbon glycoside compounds prepared in Example 23 to Example 32, and the injections of the stilbene compounds prepared in Example 33 to Example 51 were placed into a light cabinet with a illuminance of 4600 LX, and taken out and detected according to the investigation items on day 5 and 10. Test results were shown in Table 7 to Table 9.

**Table 7 Strong light exposure test results of injections of the flavonoid compounds**

| Groups | | Conditions | Properties | Related substances (total area of related substances) | pH | Content (%) |
|---|---|---|---|---|---|---|
| Control group | A sample prepared in a mixed | Before placed | Light yellow | Less than the main peak area of | 6.21 | 99.8 |
| | solvent of PEG400 and mannitol | | | control solution | | |
| | | Light exposure for 5 days | Light yellow | Less than the main peak area of control solution | 6.05 | 98.15 |
| | | Light exposure for 10 days | Light yellow | Less than the main peak area of control solution | 5.28 | 89.85 |
| Test group | Freeze-dried powder for injection of water-soluble flavonoid compounds | Before placed | Light yellow | Less than the main peak area of control solution | 6.60 | 100.65 |
| | | Light exposure for 5 days | Light yellow | Less than the main peak area of control solution | 6.54 | 99.68 |
| | | Light exposure for 10 days | Light yellow | Less than the main peak area of control solution | 6.40 | 99.01 |

**Table 8 Strong light exposure test results of injections of the carbon glycoside compounds**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Light yellow | Less than the main peak area of control solution | 6.05 | 99.5 |
| | | Light exposure for 5 days | Light yellow | Less than the main peak area of control solution | 5.05 | 95.28 |
| | | Light exposure for 10 days | Light yellow | Less than the main peak area of control solution | 4.82 | 90.55 |
| Test group | Freeze-dried powder for injection of water-soluble carbon glycoside compounds | Before placed | Light yellow | Less than the main peak area of control solution | 6.55 | 100.07 |
| | | Light exposure for 5 days | Light yellow | Less than the main peak area of control solution | 6.40 | 99.86 |
| | | Light exposure for 10 days | Light yellow | Less than the main peak area of control solution | 6.32 | 98.96 |

**Table 9 Strong light exposure test results of injections of the stilbene compounds**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Off-white | Less than the main peak area of control solution | 4.07 | 94.13 |
| | | Light exposure for 5 days | Light yellow | Less than the main peak area of control solution | 4.09 | 80.88 |
| | | Light exposure for 10 days | Light yellow | Less than the main peak area of control solution | 3.82 | 77.55 |
| Test group | Freeze-dried powder for injection of water-soluble stilbene compounds | Before placed | Light yellow | Less than the main peak area of control solution | 6.60 | 100.77 |
| | | Light exposure for 5 days | Light yellow | Less than the main peak area of control solution | 6.54 | 95.68 |
| | | Light exposure for 10 days | Light yellow | Less than the main peak area of | 6.40 | 92.69 |
| | | | | control solution | | |

Stability tests showed that the degree of degradation for the traditional sample prepared in a mixed solvent of PEG400 and mannitol was above 8%, failing to satisfy the requirements of stability for pharmaceutical preparations; while in the present invention, the injection prepared by using gastrodin as the solubilizer for the flavonoid compound, the carbon glycoside compound or the stilbene compound, as compared with that before placed, has no change in properties and related substances, and has no significant change in pH and content.

The forgoing is only for preferred embodiments of the present invention. And it should be noted that, for an ordinary skilled in the art, various improvements and modifications may be made to the present invention without departing from the principles thereof, which are deemed to fall within the protection scope of the present invention.

## Claims

1. Use of gastrodin as a solubilizer for a flavonoid compound, a carbon glycoside compound or a stilbene compound.

2. The use according to claim 1, **characterized in that** the flavonoid compound is selected from the group consisting of flavone, isoflavone, flavonol, dihydroisoflavone, dihydroflavone, isoflavane, dihydroflavonol, rotenone, anthocyanin, pterocarpin, flavan-3-ol, aurone, flavan-3,4-diol, isoaurone, chalcone, homoflavone, dihydrochalcone, phenethylchromone, xanthone or homoisoflavone.

3. The use according to claim 2, **characterized in that** the flavone is selected from the group consisting of baicalin, baicalein, wogonin or breviscapine;
the isoflavone compound is selected from the group consisting of daidzein, daidzin or puerarin;
the flavonol compound is selected from the group consisting of kaempferide or quercetin;
the dihydroflavone compound is selected from the group consisting of dihydrobaicalein, taxinine, naringenin or hesperetin;
the pterocarpin compound is maackiain; and
the homoisoflavone compound is homoisoflavone.

4. The use according to claim 1, **characterized in that** the carbon glycoside compound is selected from the group consisting of barbaloin, homoorientin, mangiferin, bergenin or aloesin.

5. The use according to claim 1, **characterized in that** the stilbene compound is selected from the group consisting of resveratrol, piceatannol, polydatin, triptophenolide, rhapontin, dexoyrhapontin, as well as a polymer formed by one or more of resveratrol, piceatannol, polydatin, triptophenolide, rhapontin and dexoyrhapontin.

6. A method of dissolving a flavonoid compound, a carbon glycoside compound or a stilbene compound by using gastrodin, **characterized in that** the method comprises the following steps:
mixing the flavonoid compound, the carbon glycoside compound or the stilbene compound with gastrodin to obtain a reactant; and
heating water for injection to 80-98 °C, followed by stirring under vacuum conditions at 30-80 °C, and then adding the reactant under the protection of nitrogen gas or argon gas to obtain a solution of the reactant.

7. The method according to claim 6, **characterized in that** the stirring time is 1~60 min.

8. The method according to claim 6, **characterized in that** the mass ratio of the flavonoid compound, the carbon glycoside compound or the stilbene compound to gastrodin is 1:0.1~80.

9. The method according to claim 6, **characterized in that** the mass ratio of the flavonoid compound, the carbon glycoside compound or the stilbene compound to gastrodin is 1:1~20.

10. The method according to claim 6, **characterized in that** the mass ratio of the flavonoid compound, the carbon glycoside compound or the stilbene compound to gastrodin is 1:3~10.

11. The method according to claim 6, **characterized in that** the mass-to-volume ratio of the gastrodin to the solution of the reactant is 2%~60% in g/mL.

12. The method according to claim 6, **characterized in that** the mass-to-volume ratio of the gastrodin to the solution of the reactant is 3%~50% in g/mL.

13. The method according to claim 6, **characterized in that** the mass-to-volume ratio of the gastrodin to the solution of the reactant is 3%~28% in g/mL.

14. A method for preparing an injection of a flavonoid compound, a carbon glycoside compound or a stilbene compound, **characterized in that** the method comprises the following steps:
mixing the flavonoid compound, the carbon glycoside compound or the stilbene compound with gastrodin to obtain a reactant;
heating water for injection to 80~98 °C, followed by stirring under vacuum conditions at 30~80 °C, and then adding the reactant under the protection of nitrogen gas or argon gas, to obtain a solution of the reactant; and adjusting pH value, filtering, filling and sterilizing to obtain the injection.

15. A method for preparing a powder for injection of a flavonoid compound, a carbon glycoside compound or a stilbene compound, **characterized in that** the method comprises the following steps:
mixing the flavonoid compound, the carbon glycoside compound or the stilbene compound with gastrodin to obtain a reactant;
heating water for injection to 80~98 °C and stirring under vacuum conditions at 30~80 °C, then adding the reactant under the protection of nitrogen gas or argon gas, to obtain a solution of the reactant; and adjusting pH value, sterile-filtering, sterile filling and freeze-drying to obtain the powder for injection.

## Patentansprüche

1. Verwendung von Gastrodin als ein Lösungsvermittler für eine Flavonoidverbindung, eine Kohlenstoffglycosidverbindung oder eine Stilbenverbindung.

2. Verwendung nach Anspruch 1, **dadurch gekennzeichnet, dass** die Flavonoidverbindung ausgewählt ist aus der Gruppe bestehend aus Flavon, Isoflavon, Flavonol, Dihydroisoflavon, Dihydroflavon, Isoflavan, Dihydroflavonol, Rotenon, Anthocyanin, Pterocarpin, Flavan-3-ol, Auron, Flavan-3,4-diol, Isoauron, Chalcon, Homoflavon, Dihydrochalcon, Phenethylchromon, Xanthon oder Homoisoflavon.

3. Verwendung nach Anspruch 2, **dadurch gekennzeichnet, dass** das Flavon ausgewählt ist aus der Gruppe bestehend aus Baicalin, Baicalein, Wogonin oder Breviscapin;
die Isoflavonverbindung ausgewählt ist aus der Gruppe bestehend aus Daidzein, Daidzin oder Puerarin;
die Flavonolverbindung ausgewählt ist aus der Gruppe bestehend aus Kaempferid oder Quercetin;
die Dihydroflavonverbindung ausgewählt ist aus der Gruppe bestehend aus Dihydrobaicalein, Taxinin, Naringenin oder Hesperetin;
die Pterocarpinverbindung Maackiain ist; und
die Homoisoflavonverbindung Homoisoflavon ist.

4. Verwendung nach Anspruch 1, **dadurch gekennzeichnet, dass** die Kohlenstoffglycosidverbindung ausgewählt ist aus der Gruppe bestehend aus Barbaloin, Homoorientin, Mangiferin, Bergenin oder Aloesin.

5. Verwendung nach Anspruch 1, **dadurch gekennzeichnet, dass** die Stilbenverbindung ausgewählt ist aus der Gruppe bestehend aus Resveratrol, Piceatannol, Polydatin, Triptophenolid, Rhapontin, Dexoyrhapontin, genauso wie einem Polymer gebildet aus einer oder mehreren von Resveratrol, Piceatannol, Polydatin, Triptophenolid, Rhapontin und Dexoyrhapontin.

6. Verfahren zum Auflösen einer Flavonoidverbindung, einer Kohlenstoffglycosidverbindung oder einer Stilbenverbindung unter Verwendung von Gastrodin, **dadurch gekennzeichnet, dass** das Verfahren die folgenden Schritte umfasst:
Mischen der Flavonoidverbindung, der Kohlenstoffglycosidverbindung oder der Stilbenverbindung mit Gastrodin um ein Reaktionsmittel zu erhalten; und
Erhitzen von Wasser zur Einspeisung auf 80~98 °C, gefolgt durch Rühren unter Vakuumbedingungen bei 30~80 °C, und dann Zugabe des Reaktionsmittels unter dem Schutz von Stickstoffgas oder Argongas, um eine Lösung des Reaktionsmittels zu erhalten.

7. Verfahren nach Anspruch 6, **dadurch gekennzeichnet, dass** die Rührzeit 1~60 min ist.

8. Verfahren nach Anspruch 6, **dadurch gekennzeichnet, dass** das Massenverhältnis der Flavonoidverbindung, der Kohlenstoffglycosidverbindung oder der Stilbenverbindnung zu Gastrodin 1:0,1~80 ist.

9. Verfahren nach Anspruch 6, **dadurch gekennzeichnet, dass** das Massenverhältnis der Flavonoidverbindung, der Kohlenstoffglycosidverbindung oder der Stilbenverbindung zu Gastrodin 1:1~20 ist.

10. Verfahren nach Anspruch 6, **dadurch gekennzeichnet, dass** das Massenverhältnis der Flavonoidverbindung, der Kohlenstoffglycosidverbindung oder der Stilbenverbindung zu Gastrodin 1:3~10 ist.

11. Verfahren nach Anspruch 6, **dadurch gekennzeichnet, dass** das Masse-zu-Volumen-Verhältnis von dem Gastrodin zu der Lösung des Reaktionsmittels 2%~60% in g/mL ist.

12. Verfahren nach Anspruch 6, **dadurch gekennzeichnet, dass** das Masse-zu-Volumen-Verhältnis von dem Gastrodin zu der Lösung des Reaktionsmittels 3%~50% in g/mL ist.

13. Verfahren nach Anspruch 6, **dadurch gekennzeichnet, dass** das Masse-zu-Volumen-Verhältnis von dem Gastrodin zu der Lösung des Reaktionsmittels 3%~28% in g/mL ist.

14. Verfahren zur Herstellung einer Injektion einer Flavonoidverbindung, einer Kohlenstoffglycosidverbindung oder einer Stilbenverbindung, dadurch gekenzeichnet, dass das Verfahren die folgenden Schritte umfasst:
Mischen der Flavonoidverbindung, der Kohlenstoffglycosidverbindung oder der Stilbenverbindung mit Gastrodin, um ein Reaktionsmittel zu erhalten;
Erhitzen von Wasser zur Einspeisung auf 80~98 °C, gefolgt durch Rühren unter Vakuumbedingungen bei 30~80 °C, und dann Zugabe des Reaktionsmittels unter dem Schutz von Stickstoffgas oder Argongas, um eine Lösung des Reaktionsmittels zu erhalten; und Anpassen des pH Wertes, Filtern, Füllen und Sterilisieren, um die Injektion zu erhalten.

15. Verfahren zur Herstellung eines Pulvers zur Injektion einer Flavonoidverbindung, einer Kohlenstoffglycosidverbindung oder einer Stilbenverbindung, **dadurch gekennzeichnet, dass** das Verfahren die folgenden Schritte umfasst:
Mischen der Flavonoidverbindung, der Kohlenstoffglycosidverbindung oder der Stilbenverbindung mit Gastrodin, um ein Reaktionsmittel zu erhalten;
Erhitzen von Wasser zur Einspeisung auf 80~98 °C und Rühren unter Vakuumbedingungen bei 30~80 °C, dann Zugabe des Reaktionsmittels unter dem Schutz von Stickstoffgas oder Argongas, um eine Lösung des Reaktionsmittels zu erhalten; und Anpassen des pH Wertes, steriles Filtern, steriles Füllen und Gefriertrocknen, um das Pulver zur Injektion zu erhalten.

## Revendications

1. Utilisation de gastrodine à titre de solubilisant pour un composé de type flavonoïde, un composé de glycoside de carbone ou un composé de stilbène.

2. Utilisation selon la revendication 1, **caractérisée en ce que** le composé de type flavonoïde est choisi dans le groupe constitué par la flavone, l'isoflavone, le flavonol, la dihydroisoflavone, la dihydroflavone, l'isoflavane, le dihydroflavonol, la roténone, l'anthocyanine, la ptérocarpine, le flavane-3-ol, l'aurone, le flavane-3,4-diol, l'isoaurone, la chalcone, l'homoflavone, la dihydrochalcone, la phénéthylchromone, la xanthone ou l'homoisoflavone.

3. Utilisation selon la revendication 2, **caractérisée en ce que** la flavone est choisie dans le groupe constitué par la baïcaline, la baïcaléine, la wogonine ou la bréviscapine ;
le composé d'isoflavone est choisi dans le groupe constitué par la daidzéine, la daidzine ou la puérarine ;
le composé de flavonol est choisi dans le groupe constitué par le kaempféride ou la quercétine ;
le composé de dihydroflavone est choisi dans le groupe constitué par la dihydrobaïcaléine, la taxinine, la naringénine ou l'hespérétine ;
le composé de ptérocarpine est la maackiaïne ; et
le composé d'homoisoflavone est l'homoisoflavone.

4. Utilisation selon la revendication 1, **caractérisée en ce que** le composé de glycoside de carbone est choisi dans le groupe constitué par la barbaloïne, l'homoorientine, la mangiférine, la bergénine ou l'aloésine.

5. Utilisation selon la revendication 1, **caractérisée en ce que** le composé de stilbène est choisi dans le groupe constitué par le resvératrol, le picéatannol, la polydatine, le triptophénolide, la rhapontine, la désoxyrhapontine, ainsi qu'un polymère formé par un ou plusieurs desdits resvératrol, picéatannol, polydatine, triptophénolide, rhapontine, et désoxy-rhapontine.

6. Procédé de dissolution d'un composé de type flavonoïde, d'un composé de glycoside de carbone ou d'un composé de stilbène à l'aide d'une gastrodine, **caractérisé en ce que** le procédé comprend les étapes suivantes :
mélange du composé de type flavonoïde, du composé de glycoside de carbone ou du composé de stilbène avec la gastrodine pour obtenir un réactif ; et
chauffage d'eau pour injection à 80~98 °C, suivi d'agitation dans des conditions de vide à 30~80 °C, puis d'addition du réactif sous protection de gaz azote ou de gaz argon pour obtenir une solution du réactif.

7. Procédé selon la revendication 6, **caractérisé en ce que** le temps d'agitation est de 1~60 min.

8. Procédé selon la revendication 6, **caractérisé en ce que** le rapport en masse du composé de type flavonoïde, du composé de glycoside de carbone ou du composé de stilbène à la gastrodine est de 1:0,1~80.

9. Procédé selon la revendication 6, **caractérisé en ce que** le rapport en masse du composé de type flavonoïde, du composé de glycoside de carbone ou du composé de stilbène à la gastrodine est de 1:1~20.

10. Procédé selon la revendication 6, **caractérisé en ce que** le rapport en masse du composé de type flavonoïde, du composé de glycoside de carbone ou du composé de stilbène à la gastrodine est de 1:3~10.

11. Procédé selon la revendication 6, **caractérisé en ce que** le rapport masse-à-volume de la gastrodine à la solution du réactif est de 2~60 % en g/mL.

12. Procédé selon la revendication 6, **caractérisé en ce que** le rapport masse-à-volume de la gastrodine à la solution du réactif est de 3~50 % en g/mL.

13. Procédé selon la revendication 6, **caractérisé en ce que** le rapport masse-à-volume de la gastrodine à la solution du réactif est de 3~28 % en g/mL.

14. Procédé de préparation d'une injection d'un composé de type flavonoïde, d'un composé de glycoside de carbone ou d'un composé de stilbène, **caractérisé en ce que** le procédé comprend les étapes suivantes :
mélange du composé de type flavonoïde, du composé de glycoside de carbone ou du composé de stilbène avec la gastrodine pour obtenir un réactif ;
chauffage d'eau pour injection à 80~98 °C, suivi d'agitation dans des conditions de vide à 30~80 °C, puis d'addition du réactif sous protection de gaz azote ou de gaz argon pour obtenir une solution du réactif ; et ajustement de la valeur de pH, filtration, remplissage et stérilisation pour obtenir l'injection.

15. Procédé de préparation d'une poudre pour injection d'un composé de type flavonoïde, d'un composé de glycoside de carbone ou d'un composé de stilbène, **caractérisé en ce que** le procédé comprend les étapes suivantes :
mélange du composé de type flavonoïde, du composé de glycoside de carbone ou du composé de stilbène avec la gastrodine pour obtenir un réactif ;
chauffage d'eau pour injection à 80~98 °C, suivi d'agitation dans des conditions de vide à 30~80 °C, puis d'addition du réactif sous protection de gaz azote ou de gaz argon pour obtenir une solution du réactif ; et ajustement de la valeur de pH, stérilisation par filtration, remplissage dans des conditions stériles et lyophilisation pour obtenir la poudre pour injection.
